# EUROPEAN PATENT SPECIFICATION

(11) **EP 1 930 404 B1**
(45) Date of publication and mention of the grant of the patent: **28.03.2012**
(21) Application number: 07022727.7
(22) Date of filing: 23.11.2007
(51) Int. Cl.: C12M 1/113, C12M 1/107

(54) **Method and device for the anaerobic fermentation of organic material**
Verfahren und Vorrichtung zur anaeroben Fermentation von organischem Material
Procédé et dispositif pour la fermentation anaérobique de matières organiques

(30) Priority: 07.12.2006 BE 200600602
(43) Date of publication of application: 11.06.2008
(73) Proprietor: Organic Waste Systems, Naamloze Vennootschap, 9000 Gent (BE)
(72) Inventor: de Baere, Luc, 9840 de Pinte (BE); Smis, Jan, 9820 Melsele (BE); Smis, Piet, 9890 Gavere (BE)
(74) Representative: Donné, Eddy

(56) References cited:
- EP-A- 1 516 921
- EP-A1- 0 038 759
- DE-U1- 29 820 752
- US-A- 5 601 720
- US-A- 5 985 149
- US-A1- 2004 164 020
- DATABASE WPI Week 200776 Derwent Publications Ltd., London, GB; AN 2007-806851 XP002474383 & CN 1 966 695 A (UNIV SOUTHERN YANGTZE) 23 May 2007 (2007-05-23)

## Description

The present invention concerns a method for the anaerobic fermentation of biodegradable organic material, whereby the fresh organic material is mixed with an amount of material which has been partly fermented as an active inoculant for the anaerobic fermentation.

The organic material is in this case biodegradable, non-liquid material, in particular various farm crops, either or not specifically cultivated for the production of energy, or the organic fraction of domestic waste, of similar industrial waste or other organic fractions, such as for example the sludge of water treatment plants, sludge of the paper industry, green waste, garden waste, organic waste flows from the production of bio-energy from farm crops, or other biodecomposable fractions comprising at least 15% of dry matter or that can be piled up.

In general, there are different ways of anaerobic fermentation. Thus, said organic flows can be fermented under wet conditions (maximally 5 to 10% dry matter in the fermentation tank) or under dry conditions (more than 15% dry matter in the fermentation tank), under either mesophilic (circa 30 to 40°C) or thermophilic (circa 50 to 60°C) conditions.

For the fermentation of organic material under wet conditions, this implies that large amounts of water are added to the organic material in order to obtain a liquid slurry in the fermentation tank of the wet systems, as a result of which the content of the fermentation tanks can be easily mixed internally and the fermented mass can be easily discharged via overflow or simply by-pumping.

For the dry systems, the amount of water is restricted to a minimum, or even no water at all is added, such that a pasty mass is obtained. This pasty mass can then be fermented in fermentation tanks that have been especially designed for dry fermentation, with a dry matter content of more than 15%. Since the high viscosity of the fermenting material in a dry fermentation with more than 15% of dry matter does not promote a smooth mixing, special mixing systems are built in in fermentation tanks that either provide for a mixing over the entire length of the fermentation tank (several mixers in the different zones or a single mixer over the entire length), or the material to be mixed is mixed outside the fermentation tank in a limited volume. Further, biogas is injected in different zones so as to produce a mixing in these zones.

In wet fermentation systems, fresh water or recycled processing water is either mixed together with the organic material and pumped into the fermentation tanks, or the organic material is pushed directly into the fermentation tank and fresh water, recycled processing water or wet, liquid organic flows are pumped into the fermentation vessel as well, whereby the ingoing mass is mixed over the entire fermentation tank so as to obtain a liquid mass. The aim hereby is always to obtain a highly liquid pulp or "slurry" that can be easily pumped and that can be easily mixed in the fermentation tank.

These wet fermentation tanks usually work according to the principle of an entirely mixed reactor whereby the ingoing mass is entirely mixed with the fermenting material over the entire volume of the fermentation tank by means of mixing gears or gas injection in the fermentation tank.

Given the liquid pulp or slurry in the fermentation tanks and the intensive internal mixing, the fermenting material and the freshly added material mix fast through the fermentation tank as a whole.

This has for a result that a part of the freshly added material will possibly be removed again from the fermentation tank within a very short time span, together with the fermented digestate. In other words, a piece of fresh food may be removed again from the fermentation tank within a time span of only a few minutes, whereas the average lingering period in the fermentation tank of the material to be fermented may amount to 20 to 30 days.

In order to solve or at least restrict this problem, as described in patent document WO 98/24730, a pre-chamber was built in that is partly separated from the rest of the entirely mixed fermentation tank by a wall. The freshly added material is first fed into this pre-chamber, which preferably has a lingering period of several days, before the material ends up in the actual fermentation space. In this way, there is already some fermentation in the pre-chamber with little risk that the supplied material will leave the fermentation tank almost immediately via the outlet together with the rest of the fermented mass as a result of the continuous mixing in the fermentation tank.

Another known solution to the above-mentioned problem is described EP 0 066 582, which represents a fermentation plant for organic materials operating with a dry matter content of 6% and at a temperature of 55 to 60°C in several reactors that are driven in an aerobic or anaerobic manner. After the anaerobic fermentation, the fermented material is normally pumped to a storage tank for the residual slurry. In a special embodiment of this device, said storage tank is driven in an anaerobic manner as an after-fermentation tank, such that additional biogas is produced from the fermented material. This requires an entirely separate additional fermentation tank, however.

Apart from the wet fermentation, as mentioned before, also dry fermentation as a method for the anaerobic fermentation of organic waste is known.

In a dry fermentation method, the amount of water that is added is limited, such that there is a relatively solid mixture in the 'dry' fermentation vessel which moves through the fermentation tank according to the principle of a sluggish flow. In order to process organic fractions of domestic waste with high contents of dry matter, for example more than 25% in the fermented digestate, an intensive mixing of the fermenting material is no longer possible in the fermentation vessel, such that the fresh organic material will have to be pre-mixed, externally to the "dry" fermentation vessel, with already fermented material by means of special mixing units. Next, the thick mixture is pumped or pushed into the fermentation vessel by means of special pumps. Other dry fermentation systems operate with contents of dry matter of up to 25% in the fermented material, which makes it possible to mix in zones by means of different mixers or in zones where gas is injected via the bottom, but only with special mixing systems. The dry fermentation tank can also be designed such that one mixer can mix the total mass with a content of dry matter of less than 25% in the fermented material when processing selectively gathered organic fractions from domestic waste. For organic fractions from domestic waste, the content of dry matter of the mixture of organic waste and fermented inoculant that is supplied to the tank is situated between 15 and 50%, more specifically between 20 and 45%. For other organic fractions such as dehydrated slurry, mixing in the fermentation tank is no longer possible as of 15 to 20%, and mixing outside the fermentation tank must already be applied as of 20% of dry matter in the fermented residue. The average content of dry matter in the dry fermentation tanks amounts to more than 15%, whereby the material is drier as it enters the fermentation tank than when it leaves the fermentation tank due to the transformation of dry matter into biogas.

EP 1.397.482 describes how this mixture of fresh organic material and fermented material is pumped into the top of a standing fermentation tank in which the fermenting mass is situated by means of risers. The supplied mixture drops from the top to the bottom during the dry fermentation, whereby the fermented material is removed from the bottom of the fermentation tank. In this way is obtained what is called a sluggish flow, whereby the material that is first supplied to the tank leaves it first as well according to the FIFO principle (First In - First Out).

In this type of dry fermentation, a sufficiently large fraction of fermented material must be mixed with the freshly supplied organic material outside the fermentor, for example five units of inoculant per unit of fresh organic material, so as to make sufficient contact between the anaerobic bacteria and the fresh organic material, since mixing is no longer possible after the mixture has been provided in the dry anaerobic fermentor.

As a major part of the fermented material is strongly recycled, the pass-through time through the fermentation tank is reduced and, depending on the recycled amount, the pass-through time will then amount to some 10 days or even 2 to 3 days. In this way, a part of freshly supplied material will be discharged together with the fermented material after 10 or even two or three days of fermentation, while the average lingering period in the fermentation tank amounts to some 12 to 30 days or more. This is already a major improvement compared to the entirely wet fermentation tanks, however, since the supplied material is guaranteed to stay 2 to 3 days or even a week in the fermentation tank, as opposed to the almost immediate removal that may occur in these easy liquid systems.

When fermenting fresh organic materials with a high production of biogas per ton, the average lingering period in the fermentation tank may increase to 50 to 100 days and more. The pass-through time only amounts to 10% or even 2 to 3% of the average lingering period then.

When the dry fermentors are heavily loaded, such as for example when fermenting high-energy crops such as maize, the fermented material, which is supposed to have fermented completely as with the known methods for anaerobic fermentation of organic material, still produces a limited amount of biogas. This represents a loss of renewable energy.

Also, when applying such known methods, germs may survive after a short pass-through time in case they are immediately removed from the fermentor and are discharged for subsequent treatment together with the rest of the digestate.

US 5.601.720 describes a continuous anaerobic fermentor process for biogenic waste, whereby control of the process is enhanced by the measurement of pH and the dry substance portions at various points in the horizontal fermentor. pH and dry substance fraction is controlled by introducing press water as inoculum at inoculation points in the fermentor.Particularly the delivery of press water laden with methanobacteria into the transporting and mixing segments of the feed pipe permits the inflowing material, consisting mostly of fresh matter, to be adjusted to the desired pH already at the fermentor entrance, thus making later corrective interventions largely unnecessary. Extensively biologically degraded material is obtained as the end product of the anaerobic fermentation.

A drawback of the previous anaerobic digestion process is that fermentation is still incomplete at the end of the process and fermentable material remains present in the final product.

The invention aims a method for the anaerobic fermentation of organic material which does not have the above-mentioned and other disadvantages.

To this aim, the method according to the invention for the anaerobic fermentation of organic material starts with organic material to be fermented which is mixed with inoculant and supplied into a fermentation tank and which moves on from an inlet of the fermentation tank to an outlet thereof, whereby the fermented material is removed from the tank via the outlet, characterized in that a fraction of the fermenting material which is situated between the inlet and the outlet is prematurely removed from the fermentation tank via a return opening is carried up to the feed device, between the inlet port and the outlet port and is used as inoculant, while the partly fermented material between the return opening and the outlet is still after-fermented for a certain while before it is removed from the fermentation tank via the outlet.

Thanks to the application of this method according to the invention, a piece of fresh organic material will stay longer in the fermentation tank as the pass-through time is extended in case such a piece of organic material is discharged to the after-treatment via the outlet after the first passage; the energetic recycling is maximized as the further produced biogas is collected; and germs are killed more efficiently.

The partly fermented material is further fermented between the return opening and the outlet for an additional length of time until a stable, fermented digestate is obtained within one and the same reactor. Thanks to this internal after-fermentation, which takes place in the fermentation tank itself, it is not necessary to provide an additional fermentation tank with accessories, which represents a serious economy. This internal after-fermentation can be applied in wet as well as dry fermentation systems, whereby the after-fermentation zone is not mixed again with the zone between the inlet and the return opening.

This also provides more certainty as to the killing of germs or weeds, since all germs or weeds that might by present have a longer pass-through time should they be immediately discharged to the after-treatment via the outlet as well.

By providing a return opening for the recycling of the inoculant, two zones are created in the fermentation tank, namely a first zone upstream of the place where a fraction of the partly fermented material is removed from the fermentation tank via the return opening, and a second zone downstream of the latter, whereby the material is removed from the second zone via the outlet for fermented digestate.

The entirely fermented digestate is usually not recycled, but possibly subjected to an after-treatment or taken directly to the fields. If, however, the fermentation would appear to be biologically less stable, an amount of fermented stable material may be added to the inoculant, i.e. the partly fermented material, so as to adjust the biological process.

In the first zone, the organically inoculated material is supplied and anaerobically fermented. A part of this fermenting material which is situated in the first zone is recycled via the return opening and mixed as an inoculant with fresh organic material to be fermented. The rest of the fermenting material ends up in the second zone downstream of the return opening.

Partly fermented material which is situated in the second zone has passed the first zone, maybe several times, as it has been recycled one or several times and has been used as inoculant. As soon as it ends up in the second zone, the partly fermented material is normally no longer fit for recycling. It steadily moves further to the outlet of the fermentation tank, preferably in a sluggish flow, depending on whether entirely fermented material is removed from the fermentation tank.

The partly fermented material is hereby subjected to an after-fermentation that occurs in this second phase in the same fermentation tank and that comes down to a mere finishing of the methanogenous phase during a period in which no additional material is being fed. The biological activity quickly decreases as the material approaches the outlet. The material stays with certainty for a certain minimum time in this second zone, and in the end it is discharged via the outlet.

Preferably, the volume of the second zone amounts to at least one fiftieth of the total volume of the fermentation tank, such that there is sufficient volume in the second zone for an after-fermentation of at least half a day to even a few days, for example 2 to 4 days or more if useful.

An additional advantage of applying such a method according to the invention is that the partly fermented material which is removed from the tank via the return opening and which is afterwards recycled and used as an inoculant, is even more biologically active than the entirely fermented material which is used as an inoculant according to the known methods.

Also the characteristics of the fermented material will have changed more than those of the partly fermented material following the after-fermentation. For example, the partly fermented material which is removed via the recycling line as an active inoculant has a pH of 7.5 to 7.8, whereas the entirely fermented material has a pH of 8.2 to 8.5. During the feeding, the pH drops to some 7, such that the acidity transition, what is called the pH shock, is less large with the partly fermented material than would be the case if the entirely fermented material were to recirculate before being mixed with the fresh organic material.

By selecting the right place for draining the inoculant via the return opening, a maximally active inoculant can be recycled, and by providing a sufficiently large volume for the after-fermentation for the partly fermented material which is not recycled as an inoculant, downstream of the return opening, it is possible to produce an optimally stabilized fermented material. Further, an optimal amount of biogas can be recycled during the after-fermentation in the two phases.

If the fermentation would begin to function less optimally from a biological point of view, part of the entirely fermented material which is discharged via the outlet could still be added to the mixture of inoculant and fresh organic material to be supplied so as to obtain an additional inoculation. In this way, the fermentation can be quickly adjusted by partly limiting the after-fermentation. The volume in the second zone may then be regarded as a reserve of intensive fermentation capacity. If necessary, it is possible to use only entirely fermented material as an inoculant with the fresh organic material to be supplied, for example so as to compensate for seasonal fluctuations or biological imbalances.

The present invention also concerns a device for implementing said method according to the invention, and to that end it comprises a fermentation tank with a fermentation room in which organic material can be fermented, a supply device which can mix fresh organic material with inoculant and can feed it through to an inlet in the fermentation tank, which is also provided with an outlet via which fermented material can be discharged, as well as with an outlet for biogas, and whereby the device is also provided with a return opening via which a fraction of the fermenting material, situated between the inlet and the outlet, can be removed from the fermentation tank and carried up to the feed device.

In order to better explain the characteristics of the invention, the following preferred embodiments of a method and device for the anaerobic fermentation of organic material according to the invention are described as an example only without being limitative in any way, with reference to the accompanying drawings, in which:
figures 1 to 3 schematically represent various embodiments of a device for the anaerobic fermentation according to the invention, seen as a section;
figure 4 is a section according to line IV-IV in figure 3;
figure 5 schematically represents an additional variant of an embodiment of a device for the anaerobic fermentation according to the invention, seen as a section.

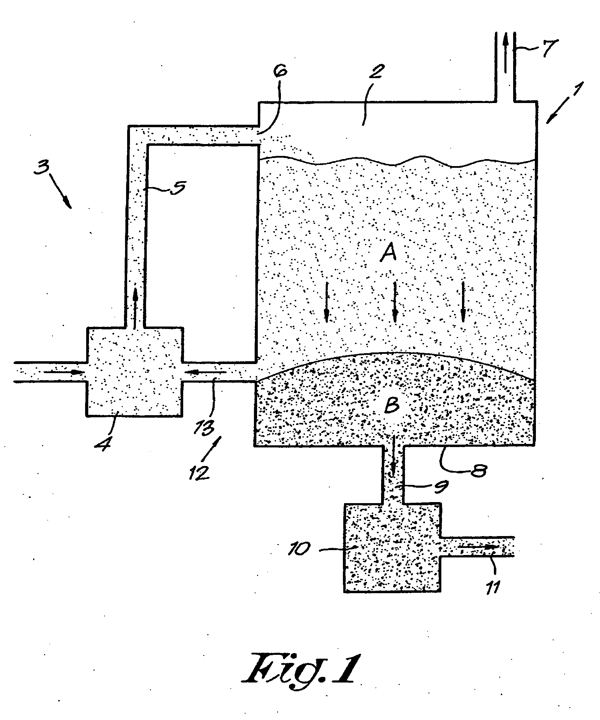

The device for the anaerobic fermentation of organic material, represented in figure 1, mainly consists of a standing, closed fermentation tank 1 comprising a fermentation room 2 and a feed device 3 which in this case consists of a mixing pump 4 for mixing fresh organic material with inoculant and for pumping this mixed mass, which in this case opens via feed lines 5 in an inlet 6 on top of the fermentation tank 1. At the top, the fermentation tank 1 is also provided with an outlet 7 for biogas.

At the bottom of the fermentation tank 1, in the center of the bottom 8, is provided a sealable outlet 9.

This outlet 9 opens in an extraction pump 10 which can discharge the entirely fermented material via lines 11 for the after-treatment.

According to the invention, the fermentation tank 1 is provided with a return opening 12 between the inlet 6 and the outlet 9 which makes it possible to discharge a fraction of the partly fermented material, situated between the inlet 6 and the outlet 9, from the fermentation tank 1 and carry it via recycling lines 13 up to the feed device 3, in this case up to the mixing pump 4, where it is mixed with freshly supplied organic material before the thus obtained mixture is put in the fermentation tank 1 via the inlet 6.

In this embodiment, the return opening 12 is situated at a height above the outlet 9 in the tank 1, in particular such that the volume occupies one fifth of the total volume of the fermentation tank 1 downstream of the return opening 12.

Two or more return openings 12 or return openings 12 that are adjustable in height can possibly be provided, such that partly fermented material can be recycled after several lingering periods.

The working of such a device, as discussed above and as represented in figure 1, as well as the method for fermenting organic material in an anaerobic manner is simple and is illustrated below by means of an example in figures.

### EXAMPLE:

Starting with a fermentation tank 1 having a total fermentation room of 2000 m³ (active volume) that is filled with fermenting organic material, 100 m³ of fresh organic material is mixed with 300 m³ of partly fermented material which is used as an inoculant during the daily feeding. It is assumed that the amount of water and/or steam supply required to obtain the desired content of dry matter in the mixing pump is equal to the tonnage of wet biogas that is produced.

In this way, 300 m³ of partly fermented material is removed daily from the fermentation tank 1 via the return opening 12 and the recycling line 13. Since the recycling line 13 opens at a height above the outlet 9 in the tank 1, the inoculant is formed of partly fermented material which is rich of active bacteria, as opposed to the organic material which is entirely decomposed when it is extracted from the fermentation tank 1 via the outlet 9, whereby the bacteria have already become significantly less active.

The fresh material and the inoculant are pushed further and are mixed by doing of the mixing pump 4.

Via the feed lines 5, the mixture is fed into the fermentation tank 1 via the inlet 6.

Thanks to an appropriate mixing ratio of the fresh organic material and the inoculant, the mixture is sufficiently pumpable and it can be pumped or carried into a closed fermentation tank. Also, the mixture is amply provided with anaerobic bacteria, such that the fermentation process may start immediately and without any notable delay.

Indeed, thanks to the fact that the inoculant is formed of partly fermented organic material which is removed from the fermentation tank at a distance from the outlet 9, the matter is bacterially active.

In the fermentation tank 1, the mixture is added to the fermenting mass and moves in the direction of the outlet 9.

Partly fermented material that is possibly further recycled via the recycling lines 13 to be used as an inoculant is situated in the zone upstream of or above the recycling lines 13, which hereafter is called the first zone A. This first zone A in this example represents a volume of some 1600 m³.

Since an average of 400 m³ of mixed material per day is pumped in at the top of the fermentation tank and the first zone A, and also 300 m³ of partly fermented material is removed from zone 1 via the recycling lines 13, and 100 m³ of entirely fermented material is removed from zone 2 at the bottom of the fermentation tank 1 via outlet 9 as well, so that 100 m³ of partly fermented material simultaneously drops through from zone 1 to zone 2, an additional lingering period of 4 days is obtained. After this average lingering period of 4 days, the provided mixture of organic material and inoculant is removed from the fermentation tank 1 via the recycling lines 13 as partly fermented material which will be added in the mixing pump 4 as inoculant.

Partly fermented material which is not removed from the fermentation tank 1 at the recycling line 13 moves further down in the direction of the outlet 9. In this zone, which is hereafter called the second zone B and which represents a volume of some 400 m³, the partly fermented material is no longer fit to be recycled. It slowly drops further in a sluggish flow to the outlet 9 of the fermentation tank 1. The material is hereby subjected to an after-fermentation that is carried out in the same fermentation tank 1 and simply comes down to the methanogenous phase being finished, whereby no additional material is being fed. The biological activity decreases as the material approaches the outlet 9. The material in this case lingers for another 4 days in this second zone B, since 100 m³ of entirely fermented material must be daily removed via outlet 9 to make room for the 100 m³ of fresh organic material that is daily added to the fermentation tank 1.

The extraction pump 10 removes the fermented material for after-treatment via the lines 11.

The average lingering period amounts to 20 days in this embodiment, since 100 m³ of fresh organic material is fed to the plant, but with an internal recycling time of the partly fermented material of 4 days in the first zone A and an after-fermentation of 4 days in the second zone B, this amounts to a minimum lingering period of 8 days for any piece of organic material that is fed to the fermentation tank.

On average, the partly fermented material is recycled 4x as an inoculant. It is possible, however, that a piece of freshly fed organic material accidentally passes the outlet as of the first time and is not recycled. This piece of organic material will then nevertheless be after-fermented for another 4 days, which amounts to a minimum lingering period of 8 days. Other pieces of organic material will be recycled 2 to 6 times and more so as to be inoculated. If no division in two zones and additional phases had been provided, a recycling time or pass-through time of 5 days would have been obtained with this fermentation tank 1.

By providing a return opening 12 and a recycling line 13, a volume of 400 m³ is created for the after-fermentation, which has for a result that the minimal guaranteed lingering period is raised from 5 to 8 days, without a second separate fermentation tank being required.

The biogas that is produced in the fermentation tank 1 is discharged via the outlet 7 for biogas that is provided at the top of the fermentation tank 1.

It is clear that the mixing pump 4 can be replaced by a mixer and a pump, or any system whatsoever to partly mix the material, and a system or device to carry the mixed material to the inlet 6 of the fermentation tank 1, or a system whereby the fresh organic material and partly fermented material are put together or are supplied to the fermentation tank 1 via a separate inlet in a specific proportion, even without any active mixing. It is also possible to install a mixer or several mixers (mechanical or with gas) in the active fermentation zone and in the ultimate fermentation zone, but in such a manner that both zones cannot be mixed, i.e. that material situated after the recycling line 13 is not mixed again with material situated in front of the recycling line 13.

It is also clear that, if in a fermentation tank 1 having the same volume of 2000 m³, the recycling lines 13 were positioned closer to the outlet 9, for example such that the first zone A occupies a volume of 1700 m³, whereas the second zone B then occupies a volume of 300 m³, and with a supply and a corresponding discharge of 150 m³ and a recycling volume of 700 m³, a recycling time of 2 days would be obtained, an after-fermentation time of 2 days and a total minimal pass-through time of 4 days, whereas the average total lingering period would then amount to 2000:150 = 13.33 days. In this way, it is possible to maintain a very high load, while nevertheless fermented material is being produced that has lingered in the fermentation tank for minimally 4 days.

It is obvious that the feed device 3 may comprise means which determine the proportion of the fresh organic material to the recycled inoculant, and that this proportion can be set or adjusted by means of a control.

It is also clear that the volumes of the zones, the recycling ratios and the average lingering periods should be adjusted and optimized depending on the organic material to be processed, the desired organic load, and the desired production of biogas as well as the stability of the partly or entirely fermented material.

Finally, it is also clear that a fermentation tank 1 according to the invention can also be provided with several return openings 12 at various distances between the inlet 6 and the outlet 9, and with accompanying recycling lines 13 going to the feed device 3, whereby an additional zone is created per additional return opening 12 between the above-mentioned zones A and B. The material in every zone has specific characteristics that can provide for a desired effect by an appropriate control of the accompanying return of organic material.

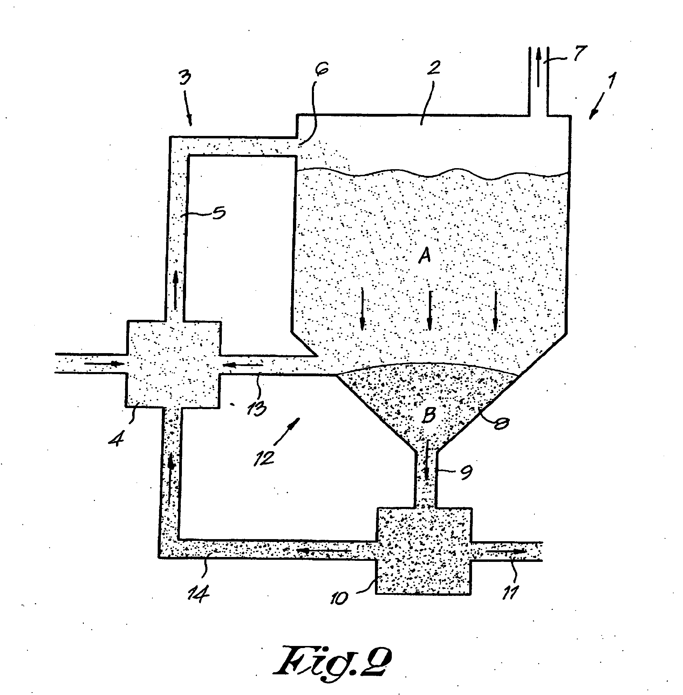

The device for the anaerobic fermentation of organic material, as represented in figure 2, differs from the above-described device in that the bottom 8 is conical in this case. The outlet 9 is connected to the lowest central point, and the recycling line 13 is connected to a higher part of the bottom 8, in particular at a height above the outlet 9.

Apart from that, this device differs from the above-described device, as represented in figure 1, in that the extraction pump 10 is provided with a return line 14 which leads up to the mixing pump 4 in this embodiment.

The working of the fermentation tank 1, as represented in figure 2, differs from the above-described fermentation tank 1, as represented in figure 1, in that the conical bottom 8 prevents a possible dead zone of fermented material in the corners near the connection of the standing walls and a flat bottom 8, as is the case in figure 1. The conical bottom may also be rounded.

The return line 14 can be used should there be any biological instability due to seasonal fluctuations, a change in quality of the waste and the like, in the first phase above the return opening 12. A partial recycling and recirculation of entirely fermented material obtained via the outlet 9, the extraction pump 10 and the return line 14 makes sure that the lingering period is extended in the first zone A and is shortened in the second zone B where the after-fermentation takes place. In an extreme case, for example only entirely fermented material would be temporarily recycled via outlet 9, such that the active fermentation would take 5 days instead of 4 days, such that a biological inhibition or temporarily additional supply of fresh organic material can be compensated for.

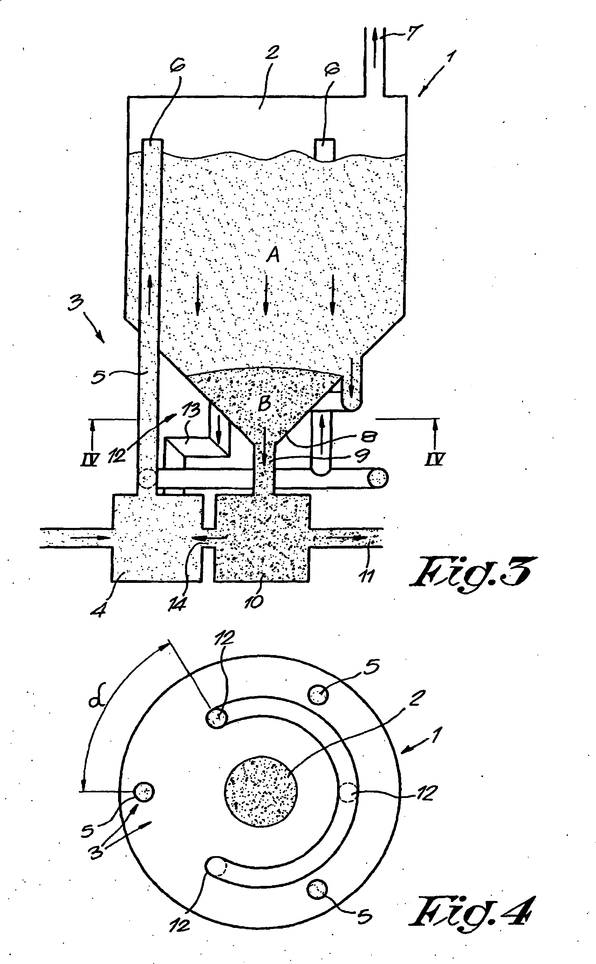

Figures 3 and 4 represent a device that differs from the above-described device for the anaerobic fermentation of organic material, as represented in figure 2, in that the feed lines 5 between the mixing pump 4 and the inlet 6 are situated partly and vertically in the fermentation tank 1. The feed lines 5 as well as the recycling lines can be partly or entirely horizontal, depending on the position of the mixing pump 4.

The outlet 12 in this case consists of several outlet points that lead to the mixing pump 4 via recycling lines 13.

Also these recycling lines 13 between the mixing pump 4 and the connection to the bottom 8 of the fermentation tank 1 run vertically, in particular up to a height above the outlet 9 in the conical part of the fermentation tank 1.

Figure 4 illustrates how three feed lines 5 are provided, here each at a mutual angular displacement of 120°, and how three recycling lines 13 are provided in a similar manner, each time one at an angular displacement α, in this case at 60° in relation to a feed line 5.

The feed lines 5 are situated near the standing wall of the fermentation tank 1 in this embodiment, whereas the recycling lines 13 are situated somewhat closer to the central outlet 9.

This can also be reversed, whereby the recycling lines 13 are built in closer to the wall, and the feed lines 5 more centrally in the conical part of the fermentation tank 1.

The supply may possibly be provided for via one or several points in the roof or at the top of the reactor via external feed lines. Further, the recycling lines can be provided higher or lower in the conical part or even in the vertical part of the wall of the fermentation tank 1, or higher in relation to the flat bottom 8 in case of a cylindrical fermentation tank 1.

The embodiment, as represented in figures 3 and 4, is advantageous in this regard in that the vertical positioning of the recycling lines 13 and of the feed lines 5, that are partly provided in the fermentation tank 1, are fed in a gravitational manner and do not require any additional space outside the tank.

Thanks to the special mutual positioning of the above-mentioned lines, well distributed and in this case at a mutual angular displacement of 60°, whereby the recycling lines are situated somewhat more centrally or more to the wall, a good flow-through of the fermenting material is obtained.

As represented in figures 3 and 4, the return opening 12 may also be formed of different outlet points. Possibly, the different outlet points are provided at mutually different distances from, or in this case at different heights above the outlet 9. Possibly, the distance of an outlet point to the outlet 9 is adjustable.

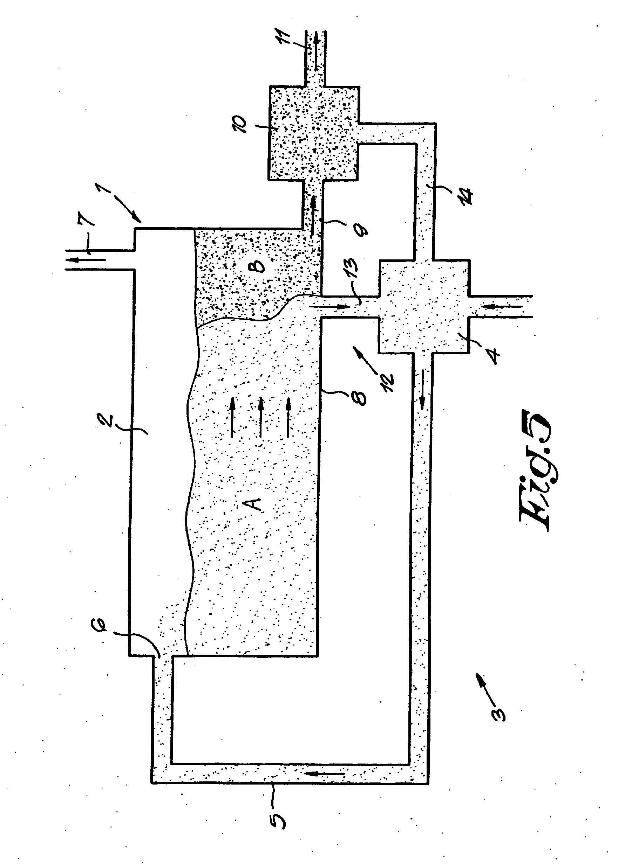

Finally, figure 5 represents a different embodiment of a device for the anaerobic fermentation of organic waste whereby the fermentation tank 1 is positioned horizontally.

The fermenting material is moved horizontally according to the principle of a sluggish flow from the inlet 6, situated on the left side in figure 5, to the outlet 9 on the right side.

The working of the fermentation tank 1, as represented in figure 5, is entirely similar to the working of the standing fermentation tank 1, as represented in figure 1, but in this embodiment the material is moved horizontally from the inlet 6 to the outlet 9.

It is clear that for all the discussed embodiments, the feed device 3 can also be built differently, and may contain for example a separate pump and mixer, or the pump may be replaced by other means to propel the fresh organic material and the inoculant in a certain proportion, possibly without any active mixer.

The fresh organic material can also be added to the fermentation tank 1 or to the feed line 5 via a separate pump, just as the partly fermented material can be added separately via another pump and feed line 5. A mixer or pusher screw can be additionally built in in the fermentation tank 1 to either mix or propel the material. Biogas can also be injected to partly propel and/or mix the fermenting material. Preferably, the horizontal push or mixing system is conceived such that there is no or only a limited mixing between the first zone A and the second zone B.

It is also clear that the return opening 12 can be connected to the feed device 3 in different ways, and that the recycling lines 13 can be replaced by other means that can provide for the transport of partly fermented material as an inoculant.

## Claims

1. Method for the anaerobic fermentation of organic material, whereby the organic material to be fermented is put in a fermentation tank (1) together with an inoculant and moves or is moved from an inlet (6) of the fermentation tank (1) to an outlet (9) thereof, whereby the fermented material is removed from the tank (1) via the outlet (9), **characterized in that** a fraction of the partly fermented material which is situated between the inlet (6) and the outlet (9) is removed from the fermentation tank (1) via at least one return opening (12) is carried up to the feed device (3) between the inlet port (6) and the outlet port (9) and is used as an inoculant, while the partly fermented material between the return opening (12) and the outlet (9) is still after-fermented for a certain while before it is removed from the fermentation tank (1) via the outlet (9).

2. Method according to claim 1, **characterized in that** the above-mentioned fraction of partly fermented material is removed via the return opening (12) after at least 12 hours and preferably after at least 2 days, and whereby partly fermented material is removed after at least 6 hours, and preferably after at least 2 days via the outlet (9).

3. Method according to claim 1, **characterized in that** a part of the fermented material which is removed from the
fermentation tank (1) via the outlet (9) is recirculated to the inlet of the fermentation tank (1), and is possibly mixed thereby with fresh organic material to be fermented and possibly also with inoculant which was removed from the fermentation tank (1) via the return opening (12).

4. Method according to any one of claims 1 to 3, **characterized in that** the material moves from top to bottom through the fermentation tank (1) in a gravitary manger.

5. Method according to claim 4, **characterized in that** there is not any mechanical mixing in the fermentation tank (1), nor any mixing with gas injection.

6. Method according to any one of claims 1 to 3, **characterized in that** the material moves horizontally from one far end to the other far end through the fermentation tank (1).

7. Device for implementing a method according to any one or several of the preceding claims, **characterized in that** it comprises a fermentation tank (1) with a fermentation room (2) in which organic material can be fermented, a feed device (3) that can mix fresh organic material with inoculant and can carry it through to an inlet (6) in the fermentation tank (1), which is also provided with an outlet (9) via which fermented material can be discharged and an outlet for biogas (7), whereby the device is also provided with a return opening (12) via which a fraction of the partly fermented material, situated between the inlet (6) and the outlet (9), can be removed from the fermentation tank (1) and can be carried up to the feed device (3).

8. Device according to claim 6, **characterized in that** the return opening (12) divides the fermentation tank (1) in a first zone (A) upstream of the return opening (12) and in a second zone (B) downstream thereof, and **in that** the return
opening (12) is situated at a sufficiently large distance from the outlet (9), preferably such that the volume of the second zone (B) occupies at least one fiftieth or even one twentieth, one fifth, one fourth, one third or up to half of the total volume of the fermentation tank (1), so as to be able to guarantee an after-fermentation of at least 6 hours and preferably of at least 2 days.

9. Device according to claim 6, **characterized in that** the feed device (3) comprises a mixing pump (4).

10. Device according to claim 6, **characterized in that** the feed device (3) comprises a mixer.

11. Device according to claim 6, **characterized in that** the feed device (3) comprises a pump.

12. Device according to claim 6, **characterized in that** the feed device (3) has two supply systems whereby fresh organic material is put in the fermentation tank (1) together with the partly fermented material or each via a separate line via a common supply line (5) or via two separate lines (5).

13. Device according to claim 6, **characterized in that** a mixing system is provided in the first zone (A).

14. Device according to claim 6, **characterized in that** a mixing system is provided in the first zone (A) and the second zone (B), whereby there is no or little mixing between the two zones. -.

15. Device according to claim 6, **characterized in that** the feed device (3) comprises means which determine the proportion of fresh organic material to recycled inoculant, and that this proportion can be set or adjusted by means of a control.

16. Device according to claim 6, **characterized in that** the feed device (3) comprises feed lines (5).

17. Device according to claim 4, **characterized in that** the fermentation tank (1) comprises two or more return openings (13).

18. Device according to claim 4, **characterized in that** the fermentation tank (1) comprises return openings (13) that can be adjusted in height.

19. Device according to claim 10, **characterized in that** the feed lines (5) open in an inlet (6) in the fermentation tank (1).

20. Device according to claim 4, **characterized in that** between the return opening (12) and the feed device (3) are provided recycling lines (13).

21. Device according to claim 4, **characterized in that** between the extraction pump (10) and the feed device (3) is provided a return line (14).

## Patentansprüche

1. Verfahren zur anaeroben Fermentation von organischem Material, wobei das zu fermentierende organische Material zusammen mit einem Impfmittel in einen Gärtank (1) eingebracht und sich von einem Einlass (6) des Gärtanks (1) zu einem Auslass (9) davon bewegt oder bewegt wird, wobei das vergorene Material durch den Auslass (9) aus dem Tank (1) entfernt wird, **dadurch gekennzeichnet, dass** eine Fraktion des teilweise vergorenen Materials, das sich zwischen dem Einlass (6) und dem Auslass (9) befindet, durch mindestens eine Rückführöffnung (12) aus dem Gärtank (1) entfernt wird, bis zu der Zuführvorrichtung (3) zwischen der Einlassöffnung (6) und der Auslassöffnung (9) befördert wird und als Impfmittel benutzt wird, während das teilweise vergorene Material zwischen der Rückführöffnung (12) und dem Auslass (9) noch für eine bestimmte Zeit nachvergoren wird, bevor es durch den Auslass (9) aus dem Gärtank (1) entfernt wird.

2. Verfahren nach Anspruch 1, **dadurch gekennzeichnet, dass** die vorgenannte Fraktion teilweise vergorenen Materials nach mindestens 12 Stunden und bevorzugt mindestens 2 Tagen durch die Rückführöffnung (12) entfernt wird, und wobei teilweise vergorenes Material nach mindestens 6 Stunden und bevorzugt mindestens 2 Tagen durch den Auslass (9) entfernt wird.

3. Vorrichtung nach Anspruch 1, **dadurch gekennzeichnet, dass** ein Teil des vergorenen Materials, das durch den Auslass (9) aus dem Gärtank (1) entfernt wird, zu dem Einlass des Gärtanks (1) rückgeführt wird und **dadurch** eventuell mit zu fermentierendem frischem organischen Material und eventuell auch mit Impfmittel, das durch die Rückführöffnung (12) aus dem Gärtank (1) entfernt wurde, gemischt wird.

4. Verfahren nach einem der Ansprüche 1 bis 3, **dadurch gekennzeichnet, dass** das Material sich durch Schwerkrafteinwirkung von oben nach unten durch den Gärtank (1) bewegt.

5. Verfahren nach Anspruch 4, **dadurch gekennzeichnet, dass** in dem Gärtank (1) weder ein mechanisches Mischen noch ein Mischen mit Gasinjektion stattfindet.

6. Verfahren nach einem der Ansprüche 1 bis 3, **dadurch gekennzeichnet, dass** sich das Material horizontal von dem einen Ende zu dem anderen Ende durch den Gärtank (1) bewegt.

7. Vorrichtung zur Verwirklichung eines Verfahrens gemäß einem oder mehreren der vorgenannten Ansprüche, **dadurch gekennzeichnet, dass** sie einen Gärtank (1) mit einem Fermentationsraum (2), worin organisches Material vergoren werden kann, eine Zuführvorrichtung (3), die frisches organisches Material mit Impfmittel vermischen kann und es zu einem Einlass (6) in dem Gärtank (1) weiterleiten kann, der ebenfalls mit einem Auslass (9), durch den vergorenes Material abgeführt werden kann, und einem Auslass für Biogas (7) versehen ist, umfasst, wobei die Vorrichtung auch mit einer Rückführöffnung (12) versehen ist, durch die eine Fraktion des teilweise vergorenen Materials, das sich zwischen dem Einlass (6) und dem Auslass (9) befindet, aus dem Gärtank (1) entfernt und bis zu der Zuführvorrichtung (3) befördert werden kann.

8. Vorrichtung nach Anspruch 6, **dadurch gekennzeichnet, dass** die Rückführöffnung (12) den Gärtank (1) in eine erste Zone (A) stromaufwärts von der Rückführöffnung (12) und in eine zweite Zone (B) stromabwärts davon unterteilt, und dass die Rückführöffnung (12) sich in einem ausreichend großen Abstand von dem Auslass (9) befindet, bevorzugt derart, dass das Volumen der zweiten Zone (B) mindestens ein Fünfzigstel oder sogar ein Zwanzigstel, ein Fünftel, ein Viertel, ein Drittel oder bis zur Hälfte des Gesamtvolumens des Gärtanks (1) einnimmt, um in der Lage zu sein, eine Nachgärung von mindestens 6 Stunden und bevorzugt von mindestens 2 Tagen zu gewährleisten.

9. Vorrichtung nach Anspruch 6, **dadurch gekennzeichnet, dass** die Zuführvorrichtung (3) eine Mischpumpe (4) umfasst.

10. Vorrichtung nach Anspruch 6, **dadurch gekennzeichnet, dass** die Zuführvorrichtung (3) eine Mischvorrichtung umfasst.

11. Vorrichtung nach Anspruch 6, **dadurch gekennzeichnet, dass** die Zuführvorrichtung (3) eine Pumpe umfasst.

12. Vorrichtung nach Anspruch 6, **dadurch gekennzeichnet, dass** die Zuführvorrichtung (3) zwei Zufuhrsysteme umfasst, wobei frisches organisches Material zusammen mit dem teilweise vergorenen Material in den Gärtank (1) eingebracht wird, oder jedes durch eine separate Leitung durch eine gemeinsame Zufuhrleitung (5) oder durch zwei separate Leitungen (5).

13. Vorrichtung nach Anspruch 6, **dadurch gekennzeichnet, dass** ein Mischsystem in der ersten Zone (A) vorgesehen ist.

14. Vorrichtung nach Anspruch 6, **dadurch gekennzeichnet, dass** ein Mischsystem in der ersten Zone (A) und der zweiten Zone (B) vorgesehen ist, wobei keine oder wenig Vermischung zwischen den zwei Zonen vorliegt.

15. Vorrichtung nach Anspruch 6, **dadurch gekennzeichnet, dass** die Zuführvorrichtung (3) Mittel umfasst, die das Verhältnis von frischem organischem Material zu wiederverwertetem Impfmittel bestimmen, und dass dieses Verhältnis mittels einer Steuerung einstellbar oder reguliert werden kann.

16. Vorrichtung nach Anspruch 6, **dadurch gekennzeichnet, dass** die Zuführvorrichtung (3) Zufuhrleitungen (5) umfasst.

17. Vorrichtung nach Anspruch 4, **dadurch gekennzeichnet, dass** der Gärtank (1) zwei oder mehr Rückführöffnungen (13) umfasst.

18. Vorrichtung nach Anspruch 4, **dadurch gekennzeichnet, dass** der Gärtank (1) Rückführöffnungen (13) umfasst, die höhenverstellbar sind.

19. Vorrichtung nach Anspruch 10, **dadurch gekennzeichnet, dass** die Zufuhrleitungen (5) in einen Einlass (6) in dem Gärtank (1) münden.

20. Vorrichtung nach Anspruch 4, **dadurch gekennzeichnet, dass** zwischen der Rückführöffnung (12) und der Zuführvorrichtung (3) Wiederverwertungsleitungen (13) vorgesehen sind.

21. Vorrichtung nach Anspruch 4, **dadurch gekennzeichnet, dass** zwischen der Extraktionspumpe (10) und der Zuführvorrichtung (3) eine Rückführleitung (14) vorgesehen ist.

## Revendications

1. Procédé pour la fermentation anaérobie d'une matière organique, la matière organique qui doit être fermentée étant placée dans une cuve de fermentation (1) conjointement avec un inoculant et se déplaçant ou étant déplacé depuis une entrée (6) de la cuve de fermentation (1) jusqu'à une sortie (9) de cette dernière, la matière fermentée étant évacuée de la cuve (1) via la sortie (9), **caractérisé en ce qu'**une fraction de la matière partiellement fermentée qui est située entre l'entrée (6) et la sortie (9) est évacuée de la cuve de fermentation (1) via au moins une ouverture de renvoi (12) entre l'orifice d'entrée (6) et l'orifice de sortie (9), est transportée jusqu'au dispositif d'alimentation (3) et est utilisée comme inoculant, tandis que la matière partiellement fermentée entre l'ouverture de renvoi (12) et la sortie (9) est toujours soumise à une fermentation ultérieure pendant un certain temps avant de l'évacuer de la cuve de fermentation (1) via la sortie (9).

2. Procédé selon la revendication 1, **caractérisé en ce que** la fraction susmentionnée de matière partiellement fermentée est évacuée via l'ouverture de renvoi (12) après au moins 12 heures et de préférence après au moins 2 jours, et la matière partiellement fermentée est évacuée après au moins 6 heures et de préférence après au moins 2 jours via la sortie (9).

3. Procédé selon la revendication 1, **caractérisé en ce qu'**une partie de la matière fermentée qui est évacuée de la cuve de fermentation (1) via la sortie (9) est remise en circulation en direction de l'entrée de la cuve de fermentation (1), pour être le cas échéant mélangée avec de la matière organique fraîche qui doit être fermentée et le cas échéant également avec l'inoculant qui doit être évacué de la cuve de fermentation (1) via l'ouverture de renvoi (12).

4. Procédé selon l'une quelconque des revendications 1 à 3, **caractérisé en ce que** la matière se déplace de bas en haut à travers la cuve de fermentation (1) sous l'effet de la pesanteur.

5. Procédé selon la revendication 4, **caractérisé en ce que** l'on ne prévoit aucun mélange mécanique dans la cuve de fermentation (1), ni aucun mélange par injection de gaz.

6. Procédé selon l'une quelconque des revendications 1 à 3, **caractérisé en ce que** la matière se déplace à l'horizontale d'une extrémité éloignée à l'autre extrémité éloignée à travers la cuve de fermentation (1).

7. Dispositif pour la mise en oeuvre d'un procédé selon une ou plusieurs des revendications précédentes, **caractérisé en ce qu'**il comprend une cuve de fermentation (1) comportant une chambre de fermentation (2) dans laquelle de la matière organique peut être fermentée ; un dispositif d'alimentation (3) qui est en mesure de mélanger de la matière organique fraîche avec l'inoculant et qui peut transporter le mélange jusqu'à une entrée (6) dans la cuve de fermentation (1) ; qui est également équipé d'une sortie (9) par laquelle la matière fermentée peut être évacuée et d'une sortie pour le biogaz (7) ; le dispositif étant également équipé d'une ouverture de renvoi (12) par laquelle une fraction de la matière partiellement fermentée, située entre l'entrée (6) et la sortie (9), peut être évacuée de la cuve de fermentation (1) et peut être transportée jusqu'au dispositif d'alimentation (3).

8. Dispositif selon la revendication 6, **caractérisé en ce que** l'ouverture de renvoi (12) subdivise la cuve de fermentation (1) en une première zone (A) en amont de l'ouverture de renvoi (12) et en une deuxième zone (B) en aval de cette dernière, et **en ce que** l'ouverture de renvoi (12) est située à une distance suffisamment grande par rapport à la sortie (9), de préférence de telle sorte que le volume de la deuxième zone (B) occupe au moins un 50e, voire un 20e, un cinquième, un quart, un tiers ou la moitié du volume total de la cuve de fermentation (1), de façon à pouvoir garantir une fermentation ultérieure d'au moins 6 heures et de préférence d'au moins 2 jours.

9. Dispositif selon la revendication 6, **caractérisé en ce que** le dispositif d'alimentation (3) comprend une pompe de mélange (4).

10. Dispositif selon la revendication 6, **caractérisé en ce que** le dispositif d'alimentation (3) comprend un mélangeur.

11. Dispositif selon la revendication 6, **caractérisé en ce que** le dispositif d'alimentation (3) comprend une pompe.

12. Dispositif selon la revendication 6, **caractérisé en ce que** le dispositif d'alimentation (3) possède deux systèmes d'alimentation, de la matière organique fraîche étant placée dans la cuve de fermentation 1 conjointement avec la matière partiellement fermentée ou, chacune, via une ligne séparée, en passant par une ligne d'alimentation commune (5) ou par deux lignes séparées (5).

13. Dispositif selon la revendication 6, **caractérisé en ce qu'**un système de mélange est prévu dans la première zone (A).

14. Dispositif selon la revendication 6, **caractérisé en ce qu'**un système de mélange est prévu dans la première zone (A) et dans la deuxième zone (B), avec une absence de mélange ou un mélange minime entre les deux zones.

15. Dispositif selon la revendication 6, **caractérisé en ce que** le dispositif d'alimentation (3) comprend un moyen qui détermine la proportion de la matière organique fraîche par rapport à l'inoculant recyclé, et **en ce que** cette proportion peut être réglée ou ajustée au moyen d'une commande.

16. Dispositif selon la revendication 6, **caractérisé en ce que** le dispositif d'alimentation (3) comprend des lignes d'alimentation (5).

17. Dispositif selon la revendication 4, **caractérisé en ce que** la cuve de fermentation (1) comprend deux ouvertures de renvoi (13) ou plus.

18. Dispositif selon la revendication 4, **caractérisé en ce que** la cuve de fermentation (1) comprend des ouvertures de renvoi (13) dont la hauteur peut être réglée.

19. Dispositif selon la revendication 10, **caractérisé en ce que** les lignes d'alimentation (5) s'ouvrent dans une entrée (6) dans la cuve de fermentation (1).

20. Dispositif selon la revendication 4, **caractérisé en ce que**, entre l'ouverture de renvoi (12) et le dispositif d'alimentation (3), on prévoit des lignes de recyclage (13).

21. Dispositif selon la revendication 4, **caractérisé en ce que**, entre la pompe d'extraction (10) et le dispositif d'alimentation (3), on prévoit une ligne de renvoi (14).
